(19) Europäisches Patentamt
European Patent Office
Office européen des brevets

(11) Veröffentlichungsnummer: **0 191 394**
**B1**

(12) **EUROPÄISCHE PATENTSCHRIFT**

(45) Veröffentlichungstag der Patentschrift: **06.12.89**

(51) Int. Cl.[4]: **C 07 F 9/165**, C 07 F 9/24, C 07 F 9/40, C 07 C 157/14

(21) Anmeldenummer: **86101376.1**

(22) Anmeldetag: **03.02.86**

(54) **Isothioharnstoffe, Verfahren zu ihrer Herstellung und ihre Verwendung zur Bekämpfung unerwünschten Pflanzenwuchses.**

(30) Priorität: **09.02.85 DE 3504452**

(43) Veröffentlichungstag der Anmeldung: **20.08.86 Patentblatt 86/34**

(45) Bekanntmachung des Hinweises auf die Patenterteilung: **06.12.89 Patentblatt 89/49**

(84) Benannte Vertragsstaaten: **BE CH DE FR GB IT LI NL**

(56) Entgegenhaltungen:
**EP-A-0 075 205**
**US-A-3 053 876**

(73) Patentinhaber: **BASF Aktiengesellschaft, Carl-Bosch- Strasse 38, D-6700 Ludwigshafen (DE)**

(72) Erfinder: **Kiehs, Karl, Dr., Sudetenstrasse 22, D-6840 Lampertheim (DE)**
Erfinder: **Würzer, Bruno, Dr., Rüdigerstrasse 13, D-6701 Otterstadt (DE)**
Erfinder: **Meyer, Norbert, Dr., Dossenheimer Weg 22, D-6802 Ladenburg (DE)**

**EP 0 191 394 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft Isothioharnstoffe, deren Herstellung, Herbizide, die diese Verbindungen als Wirkstoff enthalten, sowie deren Verwendung zur Bekämpfung unerwünschten Pflanzenwuchses.

S-alkylierte Isothioharnstoffe, die sich zur Anwendung als Herbizide eignen, sind aus der DE-OS-3 136 891 bekannt.

Daneben werden in der US-A-3 053 876 Thiophosphorsäurederivate mit pestiziden Eigenschaften insbesondere zur Bekämpfung von Mosquitolarven beschrieben.

Es wurde gefunden, daß Isothioharnstoffe der Formel I,

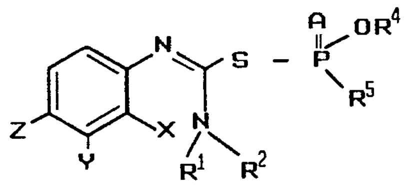

I

in der die Substituenten folgende Bedeutung haben:

X, Y und Z unabhängig voneinander Wasserstoff, Halogen, $C_1$-$C_4$-Alkyl, $C_1$-$C_4$-Alkoxy, $C_1$-$C_3$-Halogenalkyl, gegebenenfalls durch Halogen oder $C_1$-$C_4$-Alkyl substituiertes Aryloxy und/oder Aryl-$C_1$-$C_4$-alkoxy, wobei der Arylrest $C_1$-$C_4$-Alkylgruppen tragen kann,
$R^1$ $C_1$-$C_3$-Alkyl,
$R^2$ $C_1$-$C_3$-Alkyl oder Methoxy,
A ein Sauerstoff- oder Schwefelatom,
$R^4$ $C_1$-$C_4$-Alkyl, $C_3$-$C_6$-Alkoxyethyl, $C_1$-$C_3$-Halogenalkyl oder Phenyl und
$R^5$ Methyl, Ethyl, Phenyl, $C_3$-$C_6$-Alkoxyethoxy, $C_1$-$C_3$-Halogenalkoxy $C_1$-$C_4$-Alkylamino, Di-$C_1$-$C_4$-alkylamino oder $C_1$-$C_4$-Alkylthio,

herbizid sehr gut wirksam und bekannten Wirkstoffen ähnlicher Struktur überlegen sind.

In Formel I bedeuten

X, Y und Z Wasserstoff, Halogen, beispielsweise Chlor, Fluor, Brom, unverzweigtes oder verzweigtes C1-C4-Alkyl, beispielsweise Methyl, Ethyl, Isopropyl, tert.-Butyl, unverzweigtes oder verzweigtes $C_1$-$C_4$-Alkoxy, beispielsweise Methoxy, Ethoxy, i-Propoxy, unverzweigtes oder verzweigtes $C_1$-$C_3$-Halogenalkyl, beispielsweise Trifluormethyl, Aryl-$C_1$-$C_4$-alkoxy, wobei der Arylrest $C_1$-$C_4$-Alkylgruppen, beispielsweise Methylgruppen, tragen kann, beispielsweise 2-(4-Methylphenyl)-ethoxy, 2-(4-Ethylphenyl)-ethoxy, 2-(3,4-Dimethylphenyl)-ethoxy, 2-(4-n-Propylphenyl)-ethoxy, 2-(3,5-Dimethylphenyl)-ethoxy oder gegebenenfalls durch Halogen oder $C_1$-$C_4$-Alkyl substituiertes Aryloxy, beispielsweise 4-Chlorphenoxy, 1-Naphthoxy, 4-Fluorphenoxy, 4-Bromphenoxy, 3,4-Dimethylphenoxy, 4-i-Propylphenoxy, 4-t-Butylphenoxy, 3-Methyl-4-chlorphenoxy,
$R^1$ unverzweigtes oder verzweigtes $C_1$-$C_3$-Alkyl, beispielsweise Methyl, Ethyl, Isopropyl, vorzugsweise Methyl,
$R^2$ unverzweigtes oder verzweigtes $C_1$-$C_3$-Alkyl, beispielsweise Methyl, Ethyl, Isopropyl, vorzugsweise Methyl, oder Methoxy,
A ein Sauerstoff- oder Schwefelatom,
$R^4$ unverzweigtes oder verzweigtes $C_1$-$C_4$-Alkyl, $C_3$-$C_6$-Alkoxyethyl oder $C_1$-$C_3$-Halogenalkyl, wie Methyl, Ethyl, n-Butyl, n-Propyl, i-Butyl, sec.-Butyl, 2,2,2-Trichlorethyl, 2-Methoxyethyl, 2-Ethoxyethyl oder Phenyl und
$R^5$ Methyl, Ethyl, Phenyl, unverzweigte oder verzweigte $C_3$-$C_6$-Alkoxyethoxy-, $C_1$-$C_3$-Halogenalkoxy-, $C_1$-$C_4$-Alkylamino-, Di-$C_1$-$C_4$-alkylamino- oder $C_1$-$C_4$-Alkylthioreste, wie n-Propylthio, i-Propylthio, 2-Methoxy-ethoxy, 2-Ethoxy-ethoxy, 2,2,2-Trichlorethoxy, Isopropylamino, i-Butylamino, s-Butylamino, Dimethylamino, Diethylamino.

Beispiele für 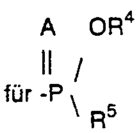 -Gruppierungen sind

O-Methyl-S-n-propyl-thiophosphoryl, O-Ethyl-S-n-propylthiophosphoryl, O-Methyl-S-i-butylthiophosphoryl, O-Methyl-S-sec-butylthiophosphoryl, O-Ethyl-S-i-butylthiophosphoryl, O-Ethyl-S-n-butylthiophosphoryl, O-Ethyl-S-sec-butylthiophosphoryl, O,O-Bis-methoxyethylphosphoryl, O,O-Bis-methoxyethylthiophosphoryl, O,O-Bis-ethoxy-ethylthiophosphoryl, O,O-Bis-ethoxyethylphosphoryl, O,O-Bis-propoxyethylthiophosphoryl, O,O-Bis-i-propoxyethylphosphoryl, O-Methyl-N-ethylamidophosphoryl, O-Ethyl-N-ethylamidophosphoryl, O-Methyl-N-i-propylamidophosphoryl, O-Methyl-N-n-propylamidophosphoryl, O-Ethyl-N-n-propylamidophosphoryl, O-Ethyl-N-i-propylamidophosphoryl, O-Ethyl-N-i-butylamidophosphoryl, O-Ethyl-N-sec-butylamidophosphoryl, O-Methyl-N,N-dimethylamidophosphoryl, O-Ethyl-N,N-dimethylami-

dophosphoryl, O-Methyl-N,N-diethylamidophosphoryl, O-Ethyl-N,N-diethylamidophosphoryl, O-Methyl-N,N-di-n-propylamidophosphoryl, O-Ethyl-N,N-di-n-propylamidophosphoryl, O-Methyl-N,N-di-n-butylamidophosphoryl, O,O-Bis-trichlorethylphosphoryl, O,O-Bis-trichlorethylthiophosphoryl, O-Methylmethylphosphonyl, O-Ethyl-methylphosphonyl, O-Methylethylphosphonyl, O-Methyl-phenylphosphonyl, O-Ethyl-phenylphosphonyl, O-Ethoxyethyl-phenylphosphonyl und O-i-Propylphenylphosphonyl.

Man erhält die Isothioharnstoffe der Formel I durch Umsetzung von Chlorformamidinen der Formel

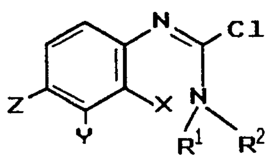

(II)

in der X, Y, Z, $R^1$ und $R^2$ die obengenannten Bedeutungen haben, mit einer Thiosäure der Formel

$$HS-\overset{\overset{A}{\|}}{P}\begin{matrix} OR^4 \\ R^5 \end{matrix} \qquad (III),$$

in Gegenwart eines säurebindenden Mittels.

Die Chlorformamidine der Formel II können durch Umsetzung von trisubstituierten Harnstoffen bzw. Thioharnstoffen mit Chlorierungsmitteln, wie $PCl_5$ bzw. $COCl_2$ erhalten werden (Houben-Weyl, Methoden der org. Chemie, Bd. E 4, S. 555 ff (1983)). Wenn $R^1$ und $R^2$ in Formel II der Chlorformamidine Alkyl, vorzugsweise Methyl, bedeuten, kann es vorteilhaft sein die Chlorformamidine ausgehend von Phosgeniminiumchlorid entsprechend folgendem Reaktionsschema herzustellen.

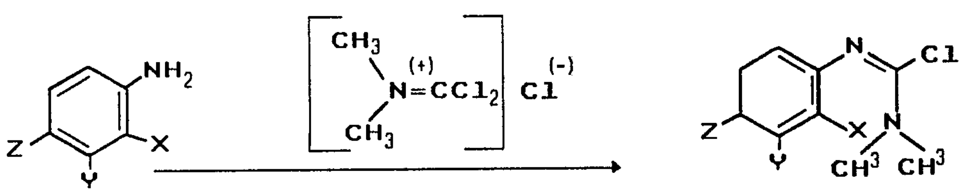

(Houben-Weyl, Methoden der org. Chemie, Bd. E 4, Seite 558 (1983)).

Die Thiosäuren der Formel III werden in Form ihrer Salze (z. B. Alkali-, Erdalkali-, Ammonium-Salze) eingesetzt, oder aber es werden die freien Säuren zusammen mit geeigneten anorganischen oder organischen Säurebindemitteln verwendet. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Beispiele hierfür sind Kaliumhydroxid, Natriumhydroxid, Kaliumcarbonat, Natriumcarbonat, Lithiumhydroxid, Lithiumcarbonat, Natriumhydrogencarbonat, Kaliumhydrogencarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumhydrogencarbonat, Magnesiumacetat, Zinkhydroxid, Zinkoxid, Zinkcarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Triamylamin, Diisopropylethylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Methylmorpholin, N-Ethylmorpholin, N-Methylhexamethylenimin, N-Ethylhexamethylenimin, Pyridin, Chinolin, α-Picolin, β-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Die Umsetzung des Chlorformamidins II mit der Thiosäure III wird mit ungefähr stöchiometrischen Substanzmengen, d.h. in einem Mengenverhältnis von etwa 1,0 bis 1,4 Verbindungen III zu 1,0 Verbindungen II durchgeführt.

Zweckmäßigerweise wird die Reaktion in einem inerten Lösungsmittel durchgeführt. In Betracht kommen z. B. Halogenkohlenwasserstoffe, insbesondere Chlorkohlenwasserstoffe, z. B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Iodbenzol, o-, p- und

m-Dichlorbenzol, o-, p-, m-Dibrombenzol, o-, m-, p-Chlortoluol, 1,2,4-Trichlorbenzol; Ether, z. B. Ethylpropylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalls von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z. B. Ethylacetat, Isobutylacetat; Ketone, z. B. Aceton, Methylethylketon: und entsprechende Gemische. Zweckmäßig verwendet man das Lösungsmittel in einer Menge von 100 bis 2000 Gew.-%, vorzugsweise von 200 bis 700 Gew.-%, bezogen auf die Ausgangsstoffe.

Die Reaktionstemperatur ist innerhalb eines größeren Bereiches variierbar. Im allgemeinen arbeitet man zwischen ungefähr 0 und ungefähr 120°C, vorzugsweise im Bereich zwischen 20 und 50°C. Da die Reaktion in einigen Fällen exotherm verläuft, kann eine Außenkühlung zu Beginn der Reaktion von Vorteil sein.

Zur Vervollständigung der Reaktion rührt man noch 15 Minuten bis 24 Stunden, vorzugsweise 1 Stunde bis 5 Stunden. Danach wird je nach Lösungsmittel direkt mit Wasser gewaschen, oder das Solvens wird abdestilliert, der Rückstand in einem mit Wasser nicht mischbaren Lösungsmittel und Wasser behandelt. In jedem Fall wird nach Phasentrennung die organische Schicht eingeengt, das als Rückstand erhaltene Produkt kann, wenn notwendig, durch Umkristallisation oder Säulenchromatographie gereinigt werden.

Die folgenden Beispiele erläutern die Herstellung der Isothioharnstoffe der Formel I.

**Beispiel**

10,4 g (0,05 mol) Phosphorpentachlorid und 11,6 g (0.05 mol) N,N-Dimethyl-N'-(3,4-dichlorphenyl)-harnstoff werden in 74 ml Toluol 3 Stunden unter Rückfluß gekocht. Nach Abdestillieren der flüchtigen Anteile bei 0,13 mbar erhält man 12,6 g N,N-Dimethyl-N'-(3,4-dichlorphenyl)-chlorformamidin in Form eines Öls; $n^{25}_D$ : 1,6133.

| | C | H | N | Cl |
|---|---|---|---|---|
| Ber. | 42,97 | 3,6 | 11,1 | 42,28 |
| Gef. | 42,7 | 3,7 | 10,6 | 42,7 |

Zu 8,8 g (0,036 mol) des Dimethylammoniumsalzes der O-Ethyl-S-n-propyldithiophosphorsäure in 75 ml Aceton tropft man bei Raumtemperatur 8,4 g (0,03 mol) N,N-Dimethyl-N'-(3,4-dichlorphenyl)-chlorformamidin. Nach 2 Stunden wird das Lösungsmittel abdestilliert und der Rückstand mit Methylenchlorid/Wasser behandelt. Durch Einengen der getrockneten organischen Phase erhält man 11,6 g N,N-Dimethyl-N'-(2,4-dichlorphenyl-S-(O-ethyl-S-n-propyl-thiophosphoryl)-isothioharnstoff als gelbes Öl; $n^{25}_D$ : 1,5943.

| | C | H | N | S | Cl | P |
|---|---|---|---|---|---|---|
| Ber. | 40,5 | 5,06 | 6,7 | 15,4 | 17,1 | 7,4 |
| Gef. | 40,9 | 5,0 | 6,8 | 14,9 | 18,6 | 7,1 |

Analog lassen sich die folgenden Isothioharnstoffe der Formel I herstellen, wobei die Gruppierung

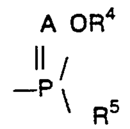

als $R^3$ in sämtlichen Tabellen aufgeführt ist.

| Verbindung | | $R^1$ | $R^2$ | $R^3$ | X | Y | ZFp [°C]/$n^{20}_n$ |
|---|---|---|---|---|---|---|---|
| 3 | $CH_3$ | $CH_3$ | $(C_2H_5O)PO(S\text{-}nC_3H_7)$ | H | Cl | Cl | 1,5943 |
| 4 | $CH_3$ | $CH_3$ | $(C_2H_5O)PO(S\text{-}nC_3H_7)$ | H | $CF_3$ | H | 1,5420 |
| 5 | $CH_3$ | $CH_3$ | $(C_2H_5O)PO(S\text{-}nC_3H_7)$ | H | H | i-$C_3H_7$ | 1,5710 |
| 6 | $CH_3$ | $CH_3$ | $(C_2H_5O)PO(S\text{-}nC_3H_7)$ | H | H | 2-(4-Methylphenyl)-ethoxy | 1,5828 |
| 7 | $CH_3$ | $OCH_3$ | $(C_2H_5O)PO(S\text{-}nC_3H_7)$ | H | Cl | Cl | 1,5885 |
| 8 | $CH_3$ | $OCH_3$ | $(C_2H_5O)PO(S\text{-}nC_3H_7)$ | H | Cl | F | |
| 9 | $CH_3$ | $OCH_3$ | $(C_2H_5O)PO(S\text{-}nC_3H_7)$ | H | F | F | |
| 29 | $CH_3$ | $CH_3$ | $C_2H_5OPO(NH\text{-}i\text{-}C_3H_7)$ | H | Cl | Cl | 1,5768 |
| 30 | $CH_3$ | $CH_3$ | $C_2H_5OPO(NH\text{-}i\text{-}C_3H_7)$ | H | $CF_3$ | H | 93 - 95 |
| 31 | $CH_3$ | $CH_3$ | $C_2H_5OPO(NH\text{-}i\text{-}C_3H_7)$ | H | H | 4-Chlor-phenoxy | 1,5900 |
| 32 | $CH_3$ | $CH_3$ | $C_2H_5OPO(NH\text{-}i\text{-}C_3H_7)$ | H | H | i-$C_3H_7$ | 1,5523 |
| 33 | $CH_3$ | $OCH_3$ | $C_2H_5OPO(NH\text{-}i\text{-}C_3H_7)$ | H | Cl | Cl | 1,5628 |

| | | | | | | | |
|---|---|---|---|---|---|---|---|
| 34 | $CH_3$ | $OCH_3$ | $C_2H_5OPO(NH\text{-}i\text{-}C_3H_7)$ | H | $CF_3$ | H | 1,5188 |
| 35 | $CH_3$ | $OCH_3$ | $C_2H_5OPO(NH\text{-}i\text{-}C_3H_7)$ | H | H | 2-(4-Methylphenyl)-ethoxy | 1,5745 |
| 36 | $CH_3$ | $CH_3$ | $C_2H_5OPO[N(CH_3)_2]$ | H | Cl | Cl | 1,5860 |
| 37 | $CH_3$ | $CH_3$ | $C_2H_5OPO[N(CH_3)_2]$ | H | $CF_3$ | H | 1,5261 |
| 38 | $CH_3$ | $OCH_3$ | $C_2H_5OPO[N(CH_3)_2]$ | H | Cl | Cl | 1,5738 |
| 39 | $CH_3$ | $OCH_3$ | $C_2H_5OPO[N(CH_3)_2]$ | H | H | $i\text{-}C_3H_7$ | |
| 40 | $CH_3$ | $OCH_3$ | $C_2H_5OPO[N(CH_3)_2]$ | F | H | F | |
| 45 | $CH_3$ | $CH_3$ | $(C_2H_5OCH_2CH_2O)_2PS$ | H | Cl | Cl | 1,5840 |
| 46 | $CH_3$ | $CH_3$ | $(C_2H_5OCH_2CH_2O)_2PS$ | H | $CF_3$ | H | 1,5283 |
| 47 | $CH_3$ | $CH_3$ | $(C_2H_5OCH_2CH_2O)_2PS$ | H | H | $i\text{-}C_3H_7$ | 1,5510 |
| 48 | $CH_3$ | $OCH_3$ | $(C_2H_5OCH_2CH_2O)_2PS$ | H | Cl | Cl | 1,5640 |
| 49 | $CH_3$ | $OCH_3$ | $(C_2H_5OCH_2CH_2O)_2PS$ | H | $CF_3$ | H | 1,5228 |
| 50 | $CH_3$ | $CH_3$ | $CH_3OPO(i\text{-}C_4H_9NH)$ | H | Cl | Cl | 1,5788 |
| 51 | $CH_3$ | $OCH_3$ | $CH_3OPO(i\text{-}C_4H_9NH)$ | H | Cl | | |
| 52 | $CH_3$ | $OCH_3$ | $CH_3OPO(i\text{-}C_4H_9NH)$ | H | $CF_3$ | H | |
| 53 | $CH_3$ | $CH_3$ | $CH_3OPO(i\text{-}C_4H_9NH)$ | H | $CF_3$ | H | |
| 54 | $CH_3$ | $CH_3$ | $CH_3OPO(i\text{-}C_4H_9NH)$ | H | H | F | |
| 55 | $CH_3$ | $CH_3$ | $(CCl_3CH_2O)_2PS$ | H | Cl | Cl | 140 - 143 |
| 56 | $CH_3$ | $OCH_3$ | $(CCl_3CH_2O)_2PS$ | H | Cl | Cl | 105 - 107 |
| 57 | $CH_3$ | $OCH_3$ | $(CCl_3CH_2O)_2PS$ | H | $CF_3$ | H | 1,5525 |
| 58 | $CH_3$ | $CH_3$ | $C_2H_5OPS(C_6H_5)$ | H | Cl | Cl | 1,6400 |
| 59 | $CH_3$ | $CH_3$ | $C_2H_5OPS(C_6H_5)$ | H | $CF_3$ | H | 1,5899 |
| 60 | $CH_3$ | $CH_3$ | $C_2H_5OPS(C_6H_5)$ | H | H | $i\text{-}C_3H_7$ | |
| 61 | $CH_3$ | $CH_3$ | $C_2H_5OPS(C_6H_5)$ | H | H | 4-Chlorphenoxy | |
| 62 | $CH_3$ | $CH_3$ | $C_2H_5OPS(C_6H_5)$ | H | H | 4-Methyl-phenoxy | |

Die Isothioharnstoffe der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, fernen Kohlenteeröle sowie Öle pflanzlichen oder tierischen Ursprungs, aliphatische, cyclische und aromatische Kohlenwasserstoffe, z. B. Toluol, Xylol, Paraffin, Tetrahydronaphthalin, alkylierte Naphthaline oder deren Derivate, z. B. Methanol, Ethanol, Propanol, Butanol, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsäure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gew.-%, vorzugsweise zwischen 0,5 und 90 Gew.-%, Wirkstoff.

**Beispiele für Formulierungen sind:**

I. Man vermischt 90 Gewichtsteile der Verbindung Nr. 29 mit 10 Gewichtsteilen N-Methyl-α-pyrrolidon und erhält eine Lösung, die zur Anwendung in Form kleinster Tropfen geeignet ist.
II. 20 Gewichtsteile des Wirkstoffs Nr. 6 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöls besteht. Durch Eingießen und feines Verteilen der Lösung in 100 000 Gewichtsteilen Wasser erhält man eine wäßrige Dispersion, die 0,02 Gew.-% des Wirkstoffs enthält.
III. 80 Gewichtsteile des Wirkstoffs Nr. 36 werden mit 3 Gewichtsteilen des Natriumsalzes der Diisobutylnaphtnalin-α-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.
IV. 5 Gewichtsteile des Wirkstoffs Nr. 50 werden mit 95 Gewichtsteilen feinteiligem Kaolin vermischt. Man erhält auf diese Weise ein Stäubemittel, das 5 Gew.-% des Wirkstoffs enthält.
V. 20 Gewichtsteile des Wirkstoffs Nr. 36 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VI. 20 Teile des Wirkstoffs Nr. 36 werden mit 2 Teilen Calciumsalz der Dodecylbenzolsulfonsäure, 2 Teilen Fettalkoholpolyglykolether, 2 Teilen Natriumsalz eines Phenol-Harnstoff-Formaldehyd-Kondensates und 68 Teile eines paraffinischen Mineralöls innig vermischt. Man erhält eine stabile ölige Dispersion.

Die Applikation der herbiziden Mittel bzw. der Wirkstoffe kann im Vorauflaufverfahren oder im Nachauflaufverfahren, vorzugsweise im Nachauflaufverfahren, erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, das die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,05 bis 5,0, vorzugsweise 0,25 bis 3,0 kg/ha.

Die herbizide Wirkung der Isothioharnstoffe der Formel I auf das Wachstum von Testpflanzen wird durch folgende Gewächshausversuche gezeigt:

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät.

Die in den Gewächshausversuchen verwendeten Pflanzen setzen sich aus folgenden Arten zusammen:

Abutilon theophrasti (Chinesischer Hanf), Amaranthus retroflexus (Zurückgekrümmter Fuchsschwanz), Arachis hypogaea (Erdnuß), Cassia tora (sicklepod), Centaurea cyanus (Kornblume), Chenopodium album (Weißer Gänsefuß), Desmodium tortuosum, Echinochloa crus-galli (Hühnerhirse), Galium aparine (Klettenlabkraut), Gossypium hirsutum (Baumwolle), Helianthus annuus (Sonnenblume), Ipomoea spp. (Prunkwindearten), Lamium amplexicaule (Stengelumfassende Taubnessel), Linum ussitatissimum (Lein), Lolium multiflorum (Ital. Raygras), Medicago sativa (Luzerne), Mercurialis annua (Einjähriges Bingelkraut), Nicandra physaloides (Gifterbse), Sesbania exaltata (Turibaum), Sinapis alba (Weißer Senf), Solanum nigrum (Schwarzer Nachtschatten), Sorghum halepense (Sudangras/Wilde Mohrenhirse), Triticum aestivum (Weizen).

Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmenge beträgt 3,0 kg Wirkstoff/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zweck der Nachauflaufbehandlung zieht man die Testpflanzen je nach Wuchsform erst bis zu einer Wuchshohe von 3 bis 15 cm an und behandelt sie danach. Es werden direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff, sie betragen beispielsweise 0,5, 1,0 oder 3 kg Wirkstoff/ha.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen verpflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Bei Vorauflaufanwendung von 3,0 kg Wirkstoff/ha zeigen beispielsweise die Verbindungen Nr. 3 und 29 eine beachtliche herbizide Aktivität, und bei 1 kg/ha ist die Verbindung 47 bei Weizen und Zuckerrüben selektiv wirksam.

**Tabelle 1** – Herbizide Aktivität bei Vorauflaufanwendung von 3,0 kg/ha am Beispiel einiger neuer Verbindungen und Testpflanzen im Gewächshaus

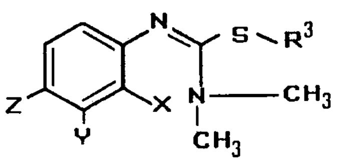

| Beispiel Nr. | $R^3$ | X | Y | Z | Testpflanzen u. Schädigung. % Echinochloa crus-galli | Lolium multifl. | Sinapis alba |
|---|---|---|---|---|---|---|---|
| 3 | 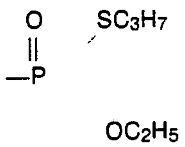 | H | Cl | Cl | 100 | 98 | 100 |
| 29 | 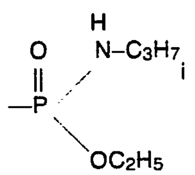 | H | Cl | Cl | 95 | 100 | 100 |

**Tabelle 2** – Bekämpfung von unerwünschten Gräsern und Kräutern in Kulturpflanzen bei Vorauflaufanwendung von Verbindung Nr. 47 im Gewächshaus

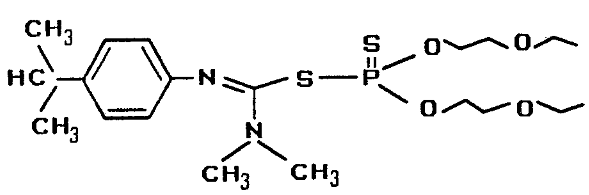

| Testpflanzen | Schädigung % 1,0 kg/ha a.S. |
|---|---|
| Beta vulgaris | 10 |
| Triticum aestivum | 0 |
| Alopecurus myosuroides | 90 |
| Avena fatua | 95 |
| Chenopodium album | 80 |

Bei Nachauflaufbehandlung erweisen sich bei der Dosierung von 3,0 kg Wirkstoff/ha die Verbindungen Nr. 30, 4, 45, 55, 48, 59 und 5 als herbizid wirksam. Breitblättrige unerwünschte Pflanzen werden selektiv in Kulturpflanzen von den Verbindungen Nr. 29, 6 und 3 mit je 1,0 kg Wirkstoff/ha bekämpft. Bei den Kulturpflanzen handelt es sich hierbei um Erdnüsse, Baumwolle, Sonnenblumen, sowie bei Anwendung von Verbindung Nr. 3 um Faserlein und Luzerne. Die selektive Bekämpfung breitblättriger Unkräuter in Erdnüssen und Weizen gelingt mit 0,5 kg Wirkstoff/ha der Verbindungen Nr. 36 und 50.

**Tabelle 3** - Herbizide Aktivität bei Nachauflaufanwendung von 3,0 kg/ha im Gewächshaus am Beispiel neuer Verbindungen und einiger Testpflanzen

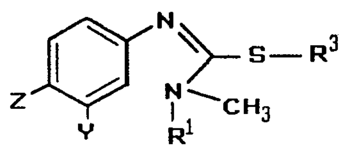

| Beisp. Nr. | $R^3$ | Y | Z | $R^1$ | Testpflanzen und Schädigung % Centaurea cyanus | Ipomoea spp. | Lolium multiflorum |
|---|---|---|---|---|---|---|---|
| 30 | 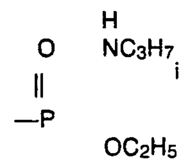 | $CF_3$ | H | $CH_3$ | 100 | 100 | 100 |
| 4 | 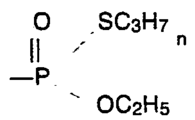 | $CF_3$ | H | $CH_3$ | 100 | 100 | 100 |
| 45 | 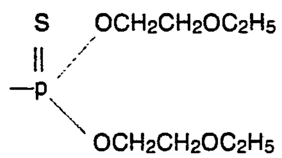 | Cl | Cl | $CH_3$ | 100 | 100 | 98 |
| 55 | 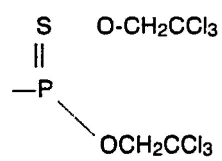 | Cl | Cl | $CH_3$ | 95 | 100 | 90 |
| 48 | 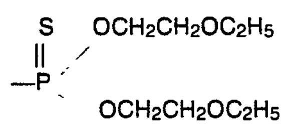 | Cl | Cl | $OCH_3$ | 95 | 100 | 90 |
| 59 | 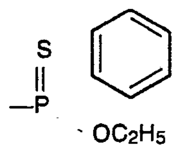 | $CF_3$ | H | $CH_3$ | 98 | 98 | 98 |
| 5 | 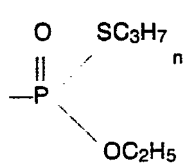 | H | $C_3H_{7i}$ | $CH_3$ | 100 | 100 | 100 |

**Tabelle 4** - Beispiel zur selektiven Unkrautbekämpfung bei Nachauflaufanwendung von Verbindung Nr. 29 im Gewächshaus

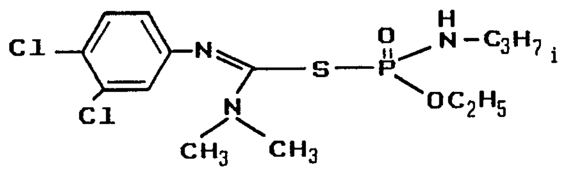

| Testpflanzen | Schädigung % bei 1,0 kg/ha a.S. |
|---|---|
| Gossypium hirsutum | 0 |
| Abutilon theophrasti | 100 |
| Sorghum halepense | 90 |

**Tabelle 5** - Selektive Bekämpfung breitblättriger Unkräuter in Sonnenblumen bei Nachauflaufanwendung von 1,0 kg/ha der Verbindung 6 im Gewächshaus

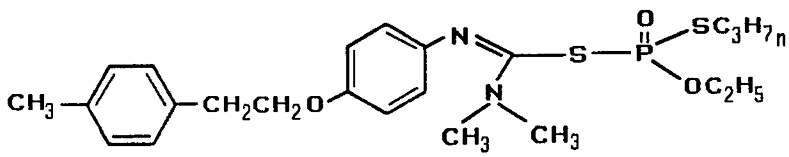

| Testpflanzen | Schädigung in % |
|---|---|
| Helianthus annuus | 10 |
| Desmodium tortuosum | 100 |
| Lamium amplex. | 100 |
| Sesbania exaltata | 80 |

**Tabelle 6** - Selektive Bekämpfung breitblättriger Unkräuter in verschiedenen Kulturen bei Nachauflaufanwendung von Verbindung Nr. 3 im Gewächshaus

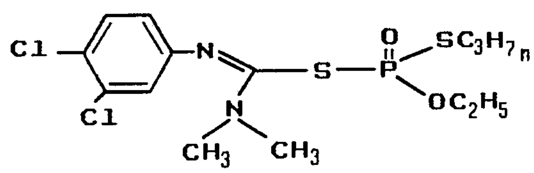

| Testpflanzen | Schädigung % bei 1,0 kg/ha a.S. |
|---|---|
| Linum ussitatissimum | 0 |
| Medicago sativa | 0 |
| Chenopodium album | 100 |
| Solanum nigrum | 100 |

**Tabelle 7** - Wirkungsbeispiel zur selektiven Unkrautbekämpfung in Erdnüssen und Weizen bei Nachauflaufanwendung im Gewächshaus

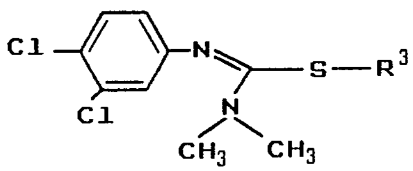

| Beispiel Nr. | $R^3$ | kg/ha a.S. | Testpflanzen und Schädigung % Arachis hypogaea | Triticum aestivum | Desmodium tortuosum | Lamium amplex. | Mercurialis annua |
|---|---|---|---|---|---|---|---|
| 50 | 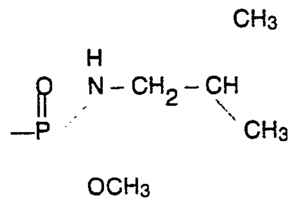 | 0,5 | 0 | 0 | 100 | 80 | 80 |
| 36 | 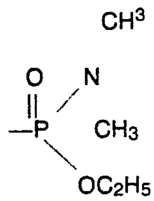 | 0,5 | 0 | 0 | 100 | 90 | 80 |

Verbindung Nr. 5 hat mit 0,5 kg Wirkstoff/ha bei Nachauflaufbehandlung eine selektive herbizide Wirkung in Weizen.

**Tabelle 8** - Beispiel zur selektiven herbiziden Wirkung vor Verbindung Nr. 5 bei Nachauflaufanwendung im Gewächshaus

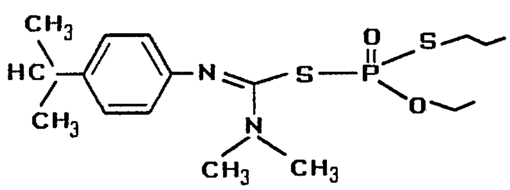

| Testpflanzen | Schädigung % bei 0,5 kg/ha a.S. |
|---|---|
| Triticum aestivum | 10 |
| Chenopodium album | 100 |
| Viola tricolor | 90 |

In Anbetracht der Vielseitigkeit der Applikationsmethoden können die Isothioharnstoffe der Formel I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| **Botanischer Name** | **Deutscher Name** |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |

| Botanischer Name | Deutscher Name |
|---|---|
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis. | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |

| Botanischer Name | Deutscher Name |
|---|---|
| Solanum tuberosum | Kartoffel |
| Sorghum bicolor (s. vulgare) | Mohrenhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais (Unterblatt oder post-directed) |

Zur Verbreiterung des Wirkungsspektrums und zur Erzielung synergistischer Effekte können die Isothioharnstoffe der Formel I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren und andere in Betracht.

Außerdem kann es von Nutzen sein, die Isothioharnstoffe der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

1. Isothioharnstoffe der Formel I

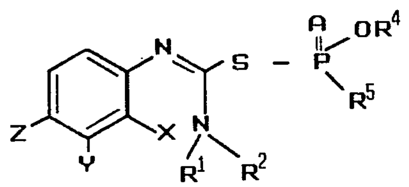

(I),

in der die Substituenten folgende Bedeutung haben:

X, Y und Z unabhängig voneinander Wasserstoff, Halogen, $C_1$-$C_4$-Alkyl, $C_1$-$C_4$-Alkoxy, $C_1$-$C_3$-Halogenalkyl, gegebenenfalls durch Halogen oder $C_1$-$C_4$-Alkyl substituiertes Aryloxy und/oder Aryl-$C_1$-$C_4$-alkoxy, wobei der Arylrest $C_1$-$C_4$-Alkylgruppen tragen kann,

$R^1$ $C_1$-$C_3$-Alkyl,
$R^2$ $C_1$-$C_3$-Alkyl oder Methoxy,
A ein Sauerstoff- oder Schwefelatom,
$R^4$ $C_1$-$C_4$-Alkyl, $C_3$-$C_6$-Alkoxyethyl, $C_1$-$C_3$-Halogenalkyl oder Phenyl und
$R^5$ Methyl, Ethyl, Phenyl, $C_3$-$C_6$-Alkoxyethoxy, $C_1$-$C_3$-Halogenalkoxy, $C_1$-$C_4$-Alkylamino, Di-$C_1$-$C_4$-alkylamino oder $C_1$-$C_4$-Alkylthio.

2. Isothioharnstoffe der Formel I gemäß Anspruch 1 *dadurch gekennzeichnet*, daß X Wasserstoff bedeutet.

3. Isothioharnstoffe der Formel I gemäß Anspruch 1, *dadurch gekennzeichnet*, daß $R^1$ Methyl und $R^2$ Methyl oder Methoxy bedeuten,

4. Verfahren zur Herstellung von Isothioharnstoffen der Formel I gemäß Anspruch 1 *dadurch gekennzeichnet*, daß man ein Chlorformamidin der Formel

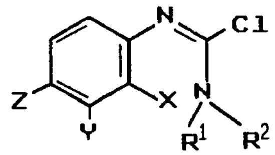

(II),

in der X, Y, Z, $R^1$ und $R^2$ die im Anspruch 1 genannten Bedeutungen haben, mit einer Thiosäure der Formel III

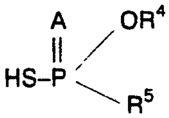

(III),

in der A, $R^4$ und $R^5$ die in Anspruch 1 genannten Bedeutungen haben in Gegenwart eines säurebindenden Mittels umsetzt.

5. Herbizid, enthaltend eine wirksame Menge Isothioharnstoff der Formel I gemäß Anspruch 1 sowie übliche Hilfsmittel.

6. Herbizid, enthaltend eine wirksame Menge Isothioharnstoff der Formel I gemäß Anspruch 2 sowie übliche Hilfsmittel.

7. Verfahren zur Bekämpfung unerwünschten Pflanzenwuchses, *dadurch gekennzeichnet*, daß man die unerwünschten Pflanzen oder die von unerwünschtem, Pflanzenwuchs freizuhaltende Fläche mit einer herbizid wirksamen Menge eines Isothioharnstoffs der Formel I gemäß Anspruch 1 behandelt.

## Claims

1. An isothiourea of the formula I

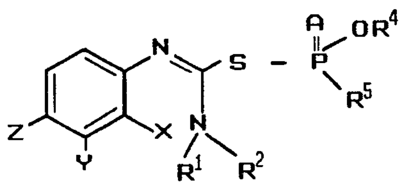

I

where
X, Y and Z independently of one another are each hydrogen, halogen, $C_1$-$C_4$-alkyl, $C_1$-$C_4$-alkoxy, $C_1$-$C_3$-haloalkyl, aryloxy which is unsubstituted or substituted by halogen or $C_1$-$C_4$-alkyl and/or aryl-$C_1$-$C_4$-alkoxy, and in which the aryl radical may,
$R^1$ is $C_1$-$C_3$-alkyl,
$R^2$ is $C_1$-$C_3$-alkyl or methoxy carry $C_1$-$C_4$-alkyl groups, A is oxygen or sulfur,
$R^4$ is $C_1$-$C_4$-alkyl, $C_3$-$C_6$-alkoxyethyl, $C_1$-$C_3$-haloalkyl or phenyl, and
$R^5$ is methyl, ethyl, phenyl, $C_3$-$C_6$-alkoxyethoxy, $C_1$-$C_3$-haloalkoxy, $C_1$-$C_4$-alkylamino, di-$C_1$-$C_4$-alkylamino or $C_1$-$C_4$-alkylthio.

2. An isothiourea of the formula I as claimed in claim 1, where X is hydrogen.

3. An isothioruea of the formula I as claimed in claim 1, where $R^1$ is methyl and $R^2$ is methyl or methoxy.

4. A process for the preparation of an isothiouria of the formula I as claimed in claim 1, wherein a chloroformamidine of the formula

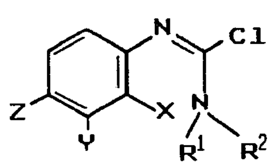

(I)

where
X, Y, Z, $R^1$ and $R^2$ have the meanings given in claim 1, is reacted with a thio acid of the formula III

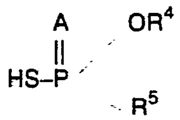

(III),

where
A, $R^4$ and $R^5$ have the meanings given in claim 1, in the presence of an acid acceptor.

5. A herbicide containing an effective amount of an isothiourea of the formula I as claimed in claim 1 and conventional auxiliaries.

6. A herbicide containing an effective amount of an isothiourea of the formula I as claimed in claim 2 and conventional auxiliaries.

7. A method for controlling undesirable plant growth, wherein the undesirable plants or the area to be kept free from undesirable plant growth are or is treated with a herbicidally effective amount of an isothiourea of the formula I as claimed in claim 1.

**Revendications**

1. Isothiourées de formule I

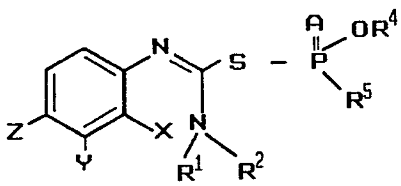

(I),

dans laquelle les symboles ont les significations suivantes:

X, Y et Z représentent chacun, indépendamment les uns des autres, l'hydrogène, un halogène, un groupe alkyle en $C_1$-$C_4$, alcoxy en $C_1$- $C_4$, halogénoalkyle en $C_1$-$C_3$, aryloxy éventuellement substitué par des halogènes ou des groupes alkyle en $C_1$-$C_4$, et/ou aryl-alcoxy en $C_1$-$C_4$ dans lequel la partie aryle peut porter des groupes alkyle en $C_1$-$C_4$,

$R^1$ représente un groupe alkyle en $C_1$-$C_3$,
$R^2$ représente un groupe alkyle en $C_1$-$C_3$ ou méthoxy,
A représente un atome d'oxygène ou de soufre,
$R^4$ représente un groupe alkyle en $C_1$-$C_4$, alcoxyéthyle en $C_3$-$C_6$, halogénoalkyle en $C_1$-$C_3$ ou phényle et
$R^5$ représente un groupe méthyle, éthyle, phényle, alcoxyethoxy en $C_3$-$C_6$, halogénoalcoxy en $C_1$-$C_3$, alkylamino en $C_1$-$C_4$, di-(alkyle en $C_1$-$C_4$)-amino ou alkylthio en $C_1$-$C_4$.

2. Isothiourées de formule I selon la revendication 1, caractérisées par le fait que X représente l'hydrogène.

3. Isothiourées de formule I selon la revendication 1, caractérisées par le fait que $R^1$ représente un groupe méthyle et $R^2$ un groupe méthyle ou méthoxy.

4. Procédé de préparation des isothiourées de formule I de la revendication 1, caractérisé par le fait que l'on fait réagir une chloroformamidine de formule II

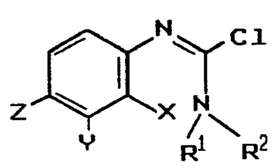

(II),

dans laquelle X, Y, Z, $R^1$ et $R^2$ ont les significations indiquées dans la revendication 1, avec un thioacide de formule III

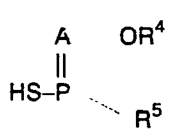

(III),

dans laquelle A, $R^4$ et $R^5$ ont les significations indiquées dans la revendication 1, en présence d'un accepteur d'acide.

5. Produit herbicide contenant une quantité efficace d'isothiourée de formule I de la revendication 1 et des produits auxiliaires usuels.

6. Produit herbicide contenant une quantité efficace d'isothiourée de formule I de la revendication 2, et des prouits auxiliaires usuels.

7. Procédé pour combattre les croissances de végétaux indesirables, caractérisé par le fait que l'on traite les végétaux indésirables ou la surface à protéger contre la croissance de végétaux indésirables par une quantité herbicide efficace d'une isothiourée de formule I de la revendication 1.